# EUROPEAN PATENT APPLICATION

(11) **EP 2 301 474 A1**
(43) Date of publication of application: **30.03.2011**
(21) Application number: 09817307.3
(22) Date of filing: 16.06.2009
(51) Int. Cl.: A61C 8/00

(54) **THREADED ELEMENT CONNECTING TO THE THREADED ORIFICE OF AN IMPLANT**

(30) Priority: 20.06.2008 ES 200801859
(71) Applicant: Biotechnology Institute, I Mas D, S.L., 01005 Vitoria (Álava) (ES)
(72) Inventor: ANITUA ALDECOA, Eduardo, E- 01005 Vitoria (ES)
(74) Representative: Urteaga Simarro, José Antonio
(86) International application number: PCT/ES2009/000333
(87) International publication number: WO 2010/037875

(57) **Abstract**

Threaded part (1), designed to be connected to a threaded hole (7) of an implant (6), which comprises a main threaded area (4) ending in a decreasing-width-provided threaded tip (5). The presence of the decreasing-width-provided threaded tip (5) has two beneficial effects: due to its decreasing width, the tip of the threaded part (1) may be inserted more easily in the threaded hole (7), even when the threaded part (1) enters the threaded hole (7) at a very pronounced angle; as it is threaded, the tip of the threaded part (1) can start to be threaded in the threaded hole (7) before the main threaded area (4), as a result of which the threaded part (1) tends to straighten itself as it is screwed in. The inventive threaded part (1) may be a prosthesis screw, a closure screw, an implant-bearing screw, a healing abutment, a transepithelial, etc.

## Description

### Technical field

The invention relates to a threaded part (and in particular to the thread system used) that is connected to an implant through its threaded hole, in order to seal or close the implant or to connect the implant to external parts such as prosthetic devices.

### Prior art

An implant is a part that is installed in the bone of a patient and whose ultimate function is to enable the connection of a prosthesis. To achieve this, the implant generally has the same approximate shape as a screw, with a head and a threaded tip. The threaded tip is designed to be connected to the bone, while the head is the area where the prosthesis and other parts involved in the surgical procedure for the installation of the implant are connected. The implant also is provided with a threaded hole designed to enable the connection of a threaded part in charge of closing or sealing the implant or of allowing the implant to be connected to external parts such as the prosthesis. For example, the threaded part may be a prosthesis screw, a closure screw, an implant-bearing screw, a healing abutment, a transepithelial, etc.

The threaded part generally used in the prior art comprises a tightening system (e.g. a groove or a cavity), a cylindrical threaded body and non-threaded cylindrical tip. The non-threaded cylindrical tip has a slightly smaller diameter than the threaded body and its purpose is to help start inserting the threaded part into the threaded hole of the implant.

The present invention aims to provide an alternative threaded part that is easier to insert in the threaded hole of the implant. It should be remembered that threaded parts for closing or sealing the implant or for connecting the implant to external parts such as prosthetic devices are generally connected to the implant once it has already been installed in the bone of the patient, and that said connection must be performed in a very small space. The insertion of said parts in the implant is usually difficult to achieve, therefore, and on occasions the surgeon may even drop the threaded part, thereby making surgery even more complex and providing additional risk to the patient.

### Brief disclosure of the invention

It is an object of this invention to provide a threaded part designed to be connected to a threaded hole of an implant, where the threaded part comprises a threaded body, with the specific characteristic that the threaded body comprises a main threaded area that ends in a threaded tip with a decreasing width. The screw thread of the descreasing-width-provided threaded tip is the same as that of the main area or is compatible with the thread of the implant. In other words, there is no obstruction when introducing one thread in the other. The presence of a descreasing-width-provided threaded tip instead of a conventional non-threaded cylindrical tip has two beneficial effects: as it has a decreasing width, the tip of the threaded part may be inserted more easily in the threaded hole, even when the threaded part enters the hole at a very pronounced angle; also, as it is threaded, the tip of the threaded part can being to be threaded into the threaded hole before the main threaded area is, as a result of which the threaded part tends to straighten itself as it is screwed in. In contrast, conventional threaded parts have to be positioned much straighter when being inserted in the hole and cannot begin to be threaded until the main threaded area is no longer in contact with the thread of the threaded hole of the implant; they may evn become jammed if the two threads are not in line.

The threaded part according to the invention may be a prosthesis screw, a closure screw, an implant-bearing screw, a healing abutment, a transepithelial, etc.

### Brief description of the drawings

Details of the invention can be seen in the accompanying non-limiting figures:
- Figure 1 shows an embodiment of the invention, where the threaded part is a healing abutment.
- Figure 2 shows a conventional healing abutment.
- Figures 3A to 3F show a sequence in which the healing abutment of Figure 1 is introduced in a threaded hole of an implant (specifically a dental implant).
- Figures 4A to 4C show a sequence in which the healing abutment of Figure 2 is introduced in a threaded hole of an implant (specifically a dental implant).
- Figure 5 shows another embodiment of a healing abutment according to the invention.

### Detailed description of the invention

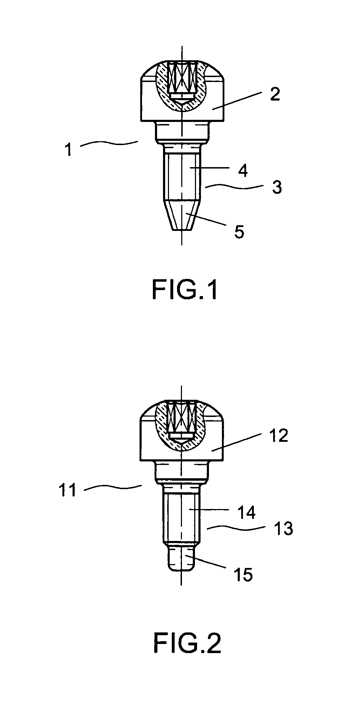

Figure 1 shows an embodiment of a threaded part according to the invention, designed to be connected to a threaded hole of a dental implant. In this embodiment, the threaded part is a healing abutment (1) designed to be connected to the implant in order to control the growth of soft tissue following surgery. The healing abutment (1) comprises a head (2) and a threaded body (3). The threaded body (3) comprises a main threaded area (4) followed by a decreasing-width-provided threaded tip (5). The screw thread of the decreasing-width-provided threaded tip (5) is preferably the same as that of the main threaded area (4); in other words, the thread between both areas (4, 5) is continuous. Alternatively, the screw thread of the decreasing-width-provided threaded tip (5) may not be identical to that of the main threaded area (4), provided that it is compatible with the thread of the implant (in other words, provided that there is no obstruction when the thread of the main threaded area (4) is inserted in the thread of the threaded hole (7) of the implant).

In the case shown in the figure, the main threaded area (4) is cylindrical although other shapes are also contemplated.

In the present embodiment, the decreasing-width-provided threaded tip (5) is conical. This solution offers a simpler and therefore cheaper manufacturing process, guaranteeing correct performance.
Figure 2 shows a conventional healing abutment. The conventional healing abutment (11) comprises a head (12) and a threaded body (13). The threaded body (13) comprises a main threaded area (14) and a non-threaded cylindrical tip (15) with a slightly smaller diameter than that of the main threaded area (14).

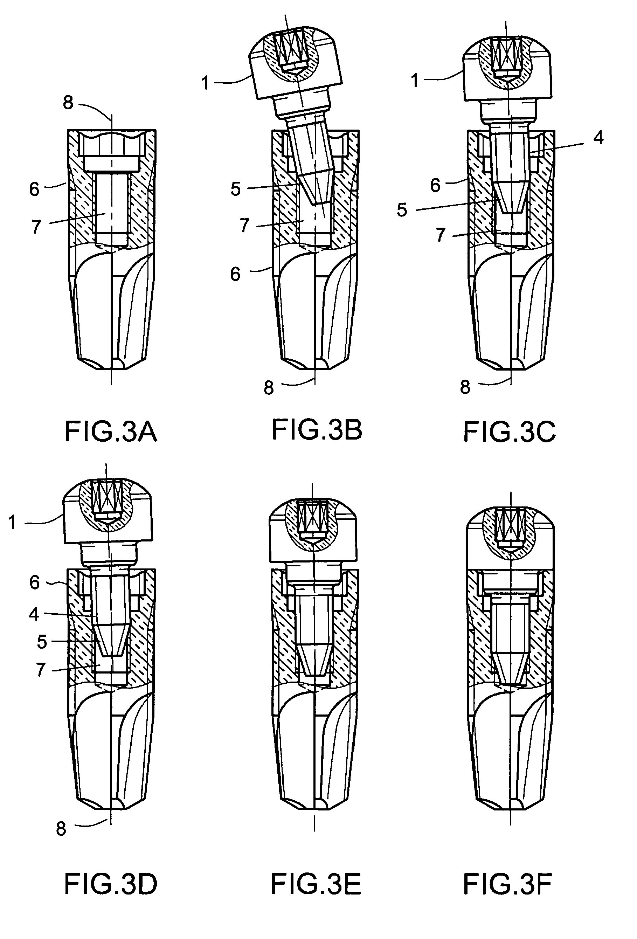

Figures 3A to 3F show a sequence in which the healing abutment (1) of Figure 1 is introduced in the threaded hole (7) of an implant (6), the implant (6) being shown in cross-section. As Figure 3B of the sequence shows, the healing abutment (1) is capable of entering at a very pronounced angle in relation to the longitudinal axis (8) of the implant (6), in spite of which the decreasing-width-provided threaded tip (5) starts thread in the inner thread of the threaded hole (7). As the healing abutment (1) is rotated it gradually straightens thanks to the conical shape of the decreasing-width-provided threaded tip (5) until finally, as can be seen in Figure 3C and even more clearly in Figure 3D, the main threaded area (4) starts to thread.

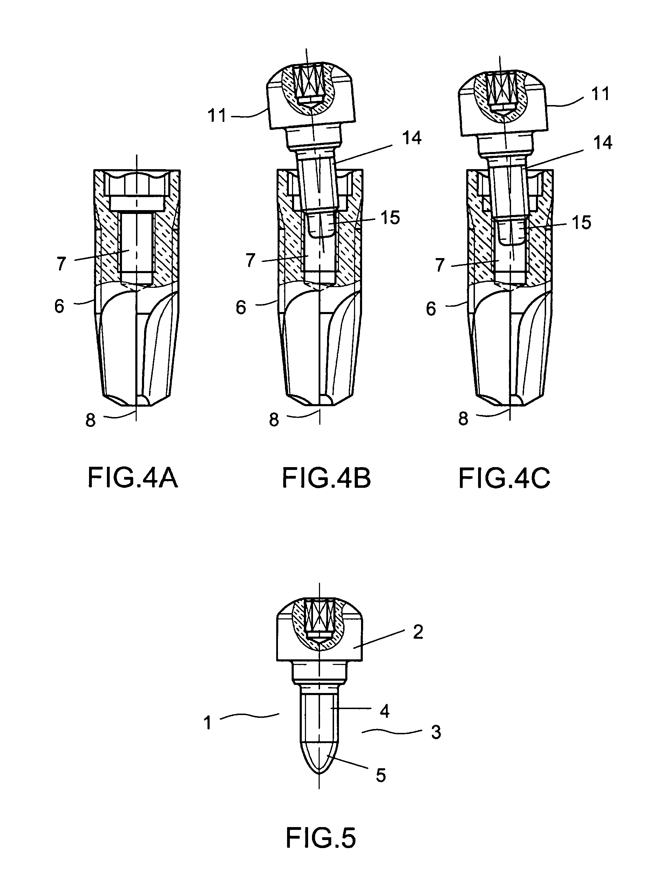

Figures 4A to 4C show a sequence in which the conventional healing abutment (1) of Figure 2 is inserted in the threaded hole (7) of an implant (6), the latter being shown in cross-section. Figure 4B shows that when the conventional healing abutment (11) is inserted at a relatively high inclination, the main threaded area (14) does not thread in the threaded hole (7) of the implant (6). The conventional healing abutment (11) needs to be straightened further, as shown in Figure 4C, so that the main threaded area (14) can start to thread in the threaded hole (7).

In other words, the healing abutment (1) according to the invention can start to thread in the threaded hole (7) of the implant (6) at a much higher inclination than the conventional healing abutment (11), as can be seen by comparing Figure 3B with Figure 4C. As a result, the healing abutment (1) according to the invention is far easier to insert in the threaded hole (7) of the implant (6) than the conventional healing abutment (11). This is a very significant advantage, particularly bearing in mind that the healing abutment must be connected to the implant located in the patient's mouth, hardly accessible, this usually being a problematic procedure.

Figure 5 shows a second embodiment of the invention. In this case the part is a healing abutment (1) wherein the decreasing-width-provided threaded tip (5) is curved.

Although the inventive threaded part shown in the figures is applied to a dental implant, it may be applied both to dental implants and other types of implants.

## Claims

1. A part (1) that is to be connected to a threaded hole (7) of an implant (6), where said part (1) comprises a threaded body (3) designed to be threaded in the threaded hole (7), **characterised in that** the threaded body (3) comprises a main threaded area (4) and a decreasing-width-provided threaded tip (5), the screw thread of the decreasing-width-provided threaded tip (5) being compatible with the screw thread of the threaded hole (7) of the implant (6).

2. Part (1) according to claim 1, wherein the main threaded area (4) is cylindrical.

3. Part (1) according to claim 1, wherein the decreasing-width-provided threaded tip (5) is entirely or partly conical.

4. Part (1) according to claim 1, wherein the decreasing-width-provided threaded tip (5) is entirely or partly curved.

5. Part (1) according to claim 1, wherein the screw thread of the decreasing-width-provided threaded tip (5) is equal to the screw thread of the main threaded area (4).
